# EUROPEAN PATENT SPECIFICATION

(11) **EP 0 448 996 B1**
(45) Date of publication and mention of the grant of the patent: **19.08.1998**
(21) Application number: 91103375.1
(22) Date of filing: 06.03.1991
(51) Int. Cl.: C07H 13/06

(54) **Process for removing soaps and fatty acids from sucrose fatty acid esters**
Verfahren zur Entfernung von Seifen und Fettsäuren aus Rohrzuckerfettsäureestern
Procédé pour éliminer des savons et des acides gras à partir d'esters gras de saccharose

(30) Priority: 08.03.1990 JP 57790/90; 28.03.1990 JP 79128/90
(43) Date of publication of application: 02.10.1991
(73) Proprietor: DAI-ICHI KOGYO SEIYAKU CO., LTD., Shimogyo-ku, Kyoto-shi, Kyoto-fu (JP)
(72) Inventor: Matsumoto, Shusaku, Fushimi-ku, Kyoto-shi (JP); Hatakawa, Yoshio, Higashiosaka-shi, Osaka-fu (JP); Nakajima, Akihiko, Nakagyo-ku, Kyoto-shi (JP)
(74) Representative: Türk, Gille, Hrabal, Leifert

(56) References cited:
- EP-A- 0 319 092
- GB-A- 1 500 341
- GB-A- 2 161 806
- US-A- 3 748 324

## Description

The present invention relates to a process for the purification of sucrose fatty acid esters (sugar esters), and more particularly to a process for decreasing the total amount of soaps and fatty acids included as impurities in the sucrose fatty acid esters.

Sucrose fatty acid esters, which are useful as surface active agents, are prepared industrially at present by either a solvent process wherein sucrose is reacted with a methyl ester of a higher fatty acid having 8 to 22 carbon atoms in the presence of a suitable catalyst in an organic solvent such as dimethylformamide or dimethylsulfoxide, as disclosed in Japanese Patent Publication Kokoku No. 35-13102; or an aqueous medium process wherein sucrose is formed into a molten mixture with a fatty acid salt (soap) using water without using an organic solvent, and is then reacted with a higher fatty acid methyl ester in the presence of a catalyst, as disclosed in Japanese Patent Publication Kokoku No. 51-14485.

In any of these processes, the obtained reaction mixture contains a relatively large amount of the unreacted sucrose, a soap, a free fatty acid and other impurities, in addition to the desired sucrose fatty acid ester. The impurities must be removed from the product prior to putting on the market. Particularly, when the sucrose fatty acid esters are used as a food additive which requires a high purity, removal of the impurities remaining in the product is very important. However, the soap and the fatty acid among the impurities included in the sucrose fatty acid ester product are particularly hard to be separated and removed, because the sucrose ester forms micelles with soap and these impurities are strongly caught therein.

The soap and the fatty acid cannot be removed from the sucrose fatty acid ester by known processes for the purification of the sucrose ester without decomposing sucrose fatty acid esters, especially sucrose fatty acid monoesters. Accordingly, the sucrose ester product industrially manufactured and put on the market at present contains always several % of these impurities.

The total amount of soap and fatty acid included in commercially available sucrose fatty acid esters is usually from 1 to 5 % by weight, and in some cases more than 5 % by weight. In order to increase the purity of the sucrose fatty acid esters, thus to raise the commercial value of the product, it has been demanded to selectively remove the soap and the fatty acid. However, no technique for efficiently removing them from the sucrose esters has been proposed.

US-A-3,748,324 discloses a process for the purification of sucrose fatty acid esters which comprises dissolving a crude reaction mixture containing the sucrose ester in a solvent comprising water and an organic solvent. This mixture is subsequently treated with at least one of the treatments (a) adding to the system an acid or an acid salt in an amount necessary to acidify the system to have a pH value within the range of 3.5 to 5.0 and recovering the sucrose ester by a liquid-liquid extraction, (b) adding to said system a salt of a metal whose valance is at least 2 to form double decomposition salts and then removing said decomposition salt to recover the sucrose ester, and (c) adding to the system a water-soluble substance to recover the sucrose ester by precipitation. However, the use of an organic solvent having a water content of at most 1.0 % by weight is not disclosed in said document.

It is a primary object of the present invention to provide a process for removing soaps and fatty acids from sucrose fatty acid esters.

The above and other objects of the present invention will become apparent from the description hereinafter.

It has been found that when oxides, hydroxides or carbonates of alkaline earth metals are added to a solution of sucrose fatty acid esters in organic solvents having a water content of at most 1.0 % by weight such as dimethylsulfoxide and dimethylformamide and stirred, the soaps (alkali metal salts of fatty acids) and the fatty acids included in the sucrose esters can be easily converted into alkaline earth metal soaps and removed as a precipitate.

In accordance with the present invention, there is provided a process for removing soaps and fatty acids from sucrose fatty acid esters which comprises adding a metal compound selected from the group consisting of calcium oxide, magnesium oxide, calcium hydroxide, magnecium hydroxide and magnesium carbonate to a solution of a sucrose fatty acid ester in an organic solvent having a water content of at most 1.0 % by weight, stirring the solution to precipitate a soap and a fatty acid, which are included in said sucrose fatty acid ester, in the form of a metal soap, and removing the resulting precipitate.

In practicing the process of the present invention, a sucrose fatty acid ester is dissolved in an organic solvent. Any organic solvents capable of dissolving the sucrose fatty acid ester can be used in the invention. Representative examples of the organic solvent are, for instance, dimethylformamide, dimethylsulfoxide and propylene glycol. The water content in the organic solvent is as low as possible, namely at most 1 % by weight. The dissolution of the sucrose ester may be carried out at ordinary temperature or an elevated temperature of not more than 100°C. The concentration of the sucrose ester is usually from 10 to 30 % by weight.

An alkaline earth metal compound is then added to the resulting solution which contains the sucrose fatty acid ester and impurities such as fatty acid and soap, at ordinary temperature or a higher temperature, preferably at an elevated temperature of not more than 100°C, with agitating the solution. The agitation is further continued. The agitation is usually conducted for 10 to 40 minutes.

During the agitation, the soap and fatty acid dissolved in the solvent are converted into a solvent-insoluble alkaline earth metal soap by the reaction represented by the formula: 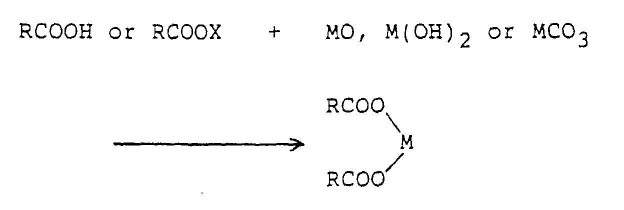 wherein X is Na or K, M is Ca or Mg and R is a fatty acid residue having 6 to 24 carbon atoms. For example, when CaO is used as the alkaline earth metal compound, the fatty acid RCOOH and the soap RCOOX are precipitated as a calcium soap Ca(RCOO)₂. When MgCO₃ is used, the fatty acid and soap are precipitated as a magnesium soap Mg(RCOO)₂.

The alkaline earth metal compounds used in the invention to precipitate the soap and fatty acid include, for instance, oxides of calcium and magnesium, hydroxides of calcium and magnesium, and magnesium carbonate. These metal compounds may be used alone or in admixture thereof. When magnesium carbonate is used with other metal compounds, a combination with magnesium hydroxide is preferred. The metal compounds are usually used in the form of a fine powder so that the reaction may be accelerated. Magnesium carbonate may be either the hydrate or anhyrous one.

The metal compounds are used usually in an amount of 20 to 200 parts by weight per 100 parts by weight of the sucrose ester.

If the organic solvent solution of the sucrose fatty acid ester contains 1 % by weight or more of water, the sucrose fatty acid ester may be hydrolyzed due to a hydrolysis acceleration action of calcium or magnesium metal. Fatty acid monoesters of sucrose are hydrolyzed in preference to fatty acid diesters and triesters of sucrose. Accordingly it is necessary to maintain the water content in the solution of the sucrose ester below 1 % by weight.

The fatty acid and soap which have been precipitated as calcium or magnesium metal soap by the above procedure, can be easily separated and removed as a cake in a usual manner, e.g. filtration or centrifugation, using a usual separation device. The sucrose fatty acid ester wherein the total amount of soap and fatty acid is decreased, is obtained by removing the organic solvent from the resulting filtrate, for example, by distilling away the organic solvent from the filtrate undr reduced pressure. For example, when dimethylsulfoxide (DMSO) is used as the organic solvent, the filtrate is subjected to distillation at a temperature of 80° to 100°C under vacuum of 666.6 to 1333.2 Pa (5 to 10 Torrs), whereby sucrose fatty acid ester containing a decreased amount of the soap and fatty acid is obtained as the residue.

If it is desired to further decrease the total amount of the soap and the fatty acid in the sucrose ester, the above procedures of the dissolution of sucrose ester, the addition of metal compound, the agitation of solution and the separation of precipitate may be completely or partially repeated.

According to the process of the present invention, the total content of an alkali metal soap and a fatty acid, which are usually contained in a sucrose fatty acid ester in an amount of 1 to 5 % by weight, can be decreased to about 0.1 to about 3.0 % by weight. Thus, the purity of the sucrose ester is improved, and also it is expected that it gives an improved taste because of decrease of soap and fatty acid.

The present invention is more specifically described and explained by means of the following Examples in which all % are by weight unless otherwise noted. It is to be understood that the present invention is not limited to the Examples.

### Example 1

A sucrose fatty acid ester produced by a reaction of sucrose and a fatty acid methyl ester mixture of methyl stearate and methyl palmitate in a ratio of 7 : 3 by weight and having the following composition was purified.

| (Composition of Sucrose Ester) | |
|---|---|
| Ingredients | Amounts (%) |
| Sucrose fatty acid ester* | 94.6 |
| Unreacted fatty acid methyl esters and others | 0.9 |
| Soap | 3.5 |
| Fatty acid | 1.0 |
| Total | 100.0 |

| | |
|---|---|
| (Note) * The sucrose ester had a monoester content 70.5 %, a diester content 19.2 % and a triester and higher-ester content 10.3 %. | |

To 100 g of dimethylsulfoxide (DMSO) having a water content of 0.4 % was added 25 g of the sucrose fatty acid ester having the above composition, and the mixture was stirred at 90°C for 20 minutes to dissolve the sucrose ester. To the resulting solution was added 15.0 g of CaO powder, Ca(OH)₂ powder, MgO powder or Mg(OH)₂ powder, and the mixture was stirred at 90°C for 10 minutes. The mixture was then filtered, and dimethylsulfoxide was distilled away from the filtrate to give purified sucrose fatty acid ester. The contents of soap and fatty acid in the purified sucrose ester free of DMSO are shown in Table 1.

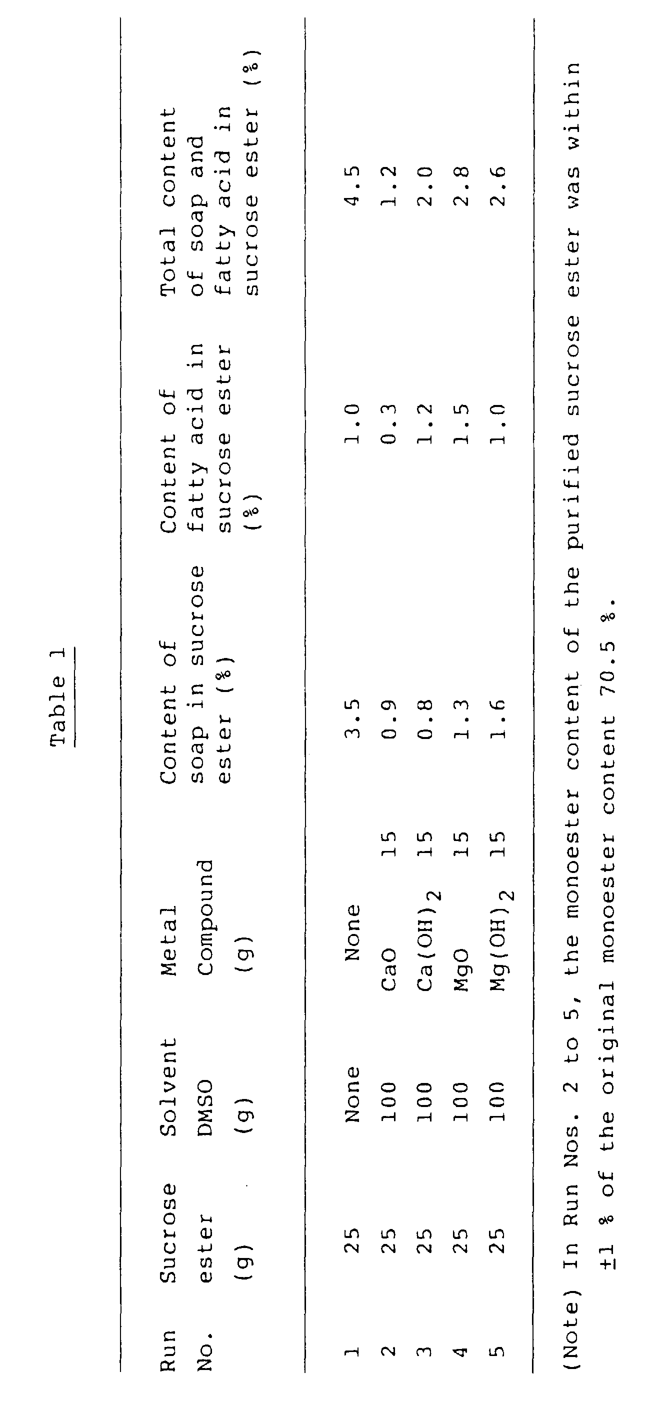

### Example 2

The procedure of Example 1 was repeated except that a sucrose fatty acid ester having the composition shown below was used.

| (Composition of Sucrose Ester) | |
|---|---|
| Ingredients | Amounts (%) |
| Sucrose fatty acid ester* | 96.9 |
| Unreacted fatty acid methyl esters and others | 1.5 |
| Soap | 1.1 |
| Fatty acid | 0.5 |
| Total | 100.0 |

| | |
|---|---|
| (Note) * The sucrose ester had a monoester content 53.2 %, a diester content 35.3 % and a triester and higher-ester content 11.5 %. | |

The contents of soap and fatty acid in the purified sucrose ester free of DMSO are shown in Table 2.

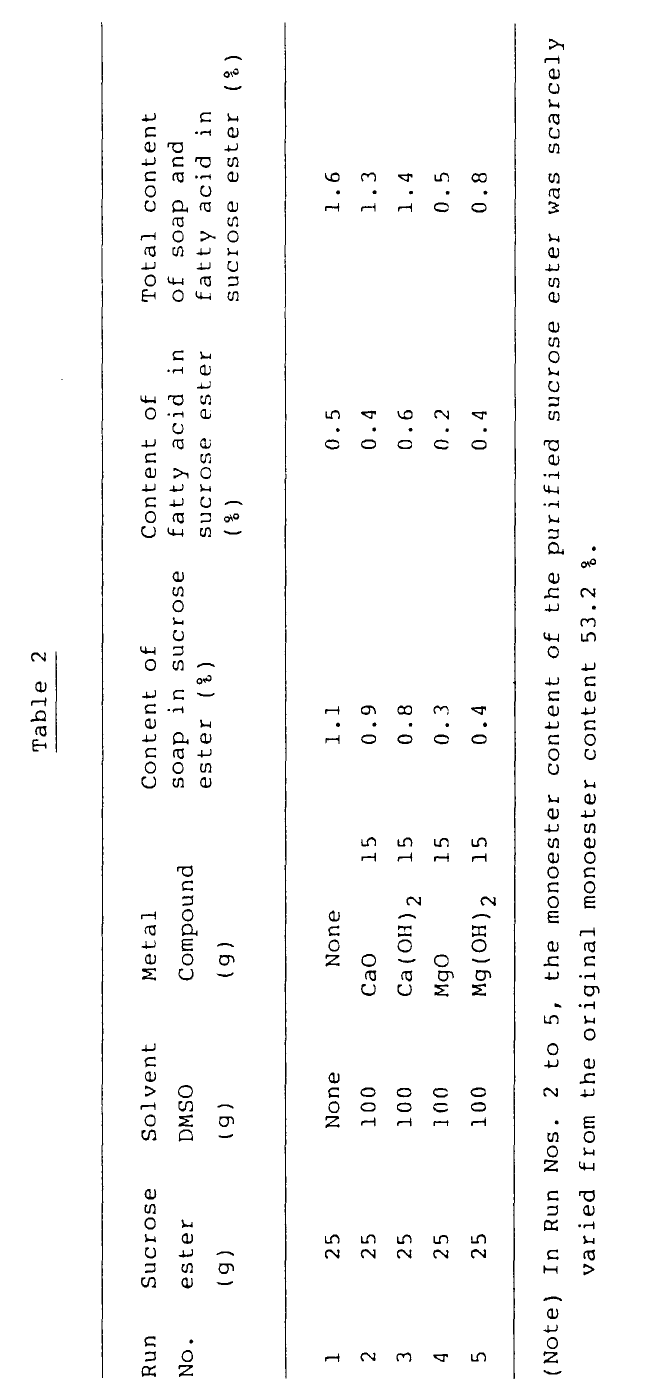

### Example 3

To 150 g of dimethylsulfoxide (DMSO) having a water content of 0.6 % was added 25 g the same sucrose fatty acid ester as used in Example 1, and the mixture was stirred at 80°C for 10 minutes to dissolve the sucrose ester. To the resulting solution was added 25.0 g of MgCO₃ powder, and the mixture was stirred at 80°C for 30 minutes. The resulting precipitate was filtered off, and dimethylsulfoxide was distilled away from the filtrate to give purified sucrose fatty acid ester. The contents of soap and fatty acid in the purified sucrose ester free of DMSO are shown in Table 3.

### Example 4

The procedure of Example 3 was repeated except that 18.0 g of MgCO₃ powder and 7.0 g of Mg(OH)₂ powder (total 25.0 g) were used instead of 25.0 g of MgCO₃ powder.

The result is shown in Table 3.

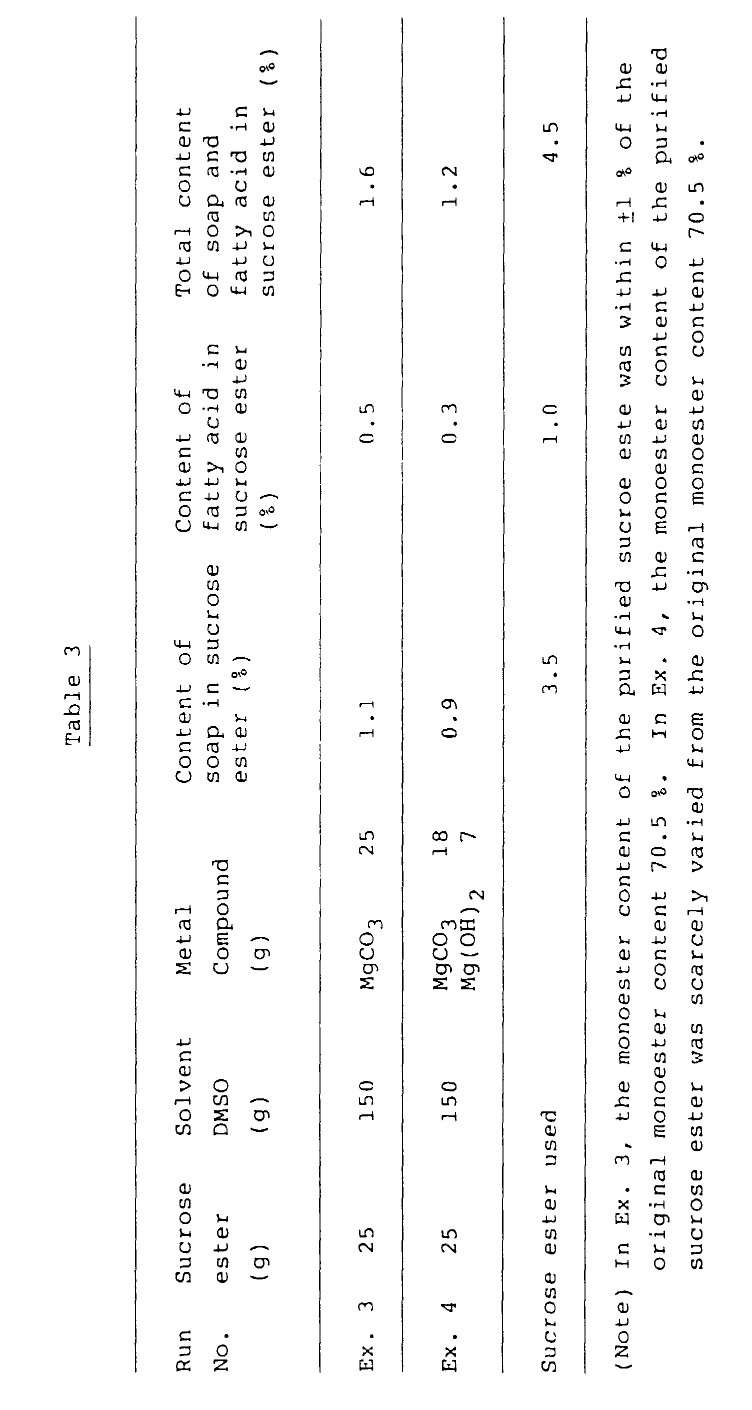

### Example 5

The procedure of Example 3 was repeated except that a sucrose fatty acid ester having the composition shown below was used.

| (Composition of Sucrose Ester) | |
|---|---|
| Ingredients | Amounts (%) |
| Sucrose fatty acid ester* | 96.2 |
| Unreacted fatty acid methyl esters and others | 1.6 |
| Soap | 1.4 |
| Fatty acid | 0.8 |
| Total | 100.0 |

| | |
|---|---|
| (Note) * The sucrose ester had a monoester content 53.3 %, a diester content 35.0 % and a triester and higher-ester content 11.7 %. | |

The contents of soap and fatty acid in the purified sucrose ester free of DMSO are shown in Table 4.

In addition to the ingredients used in the Examples, other ingredients can be used in the Examples as set forth in the specification to obtain substantially the same results.

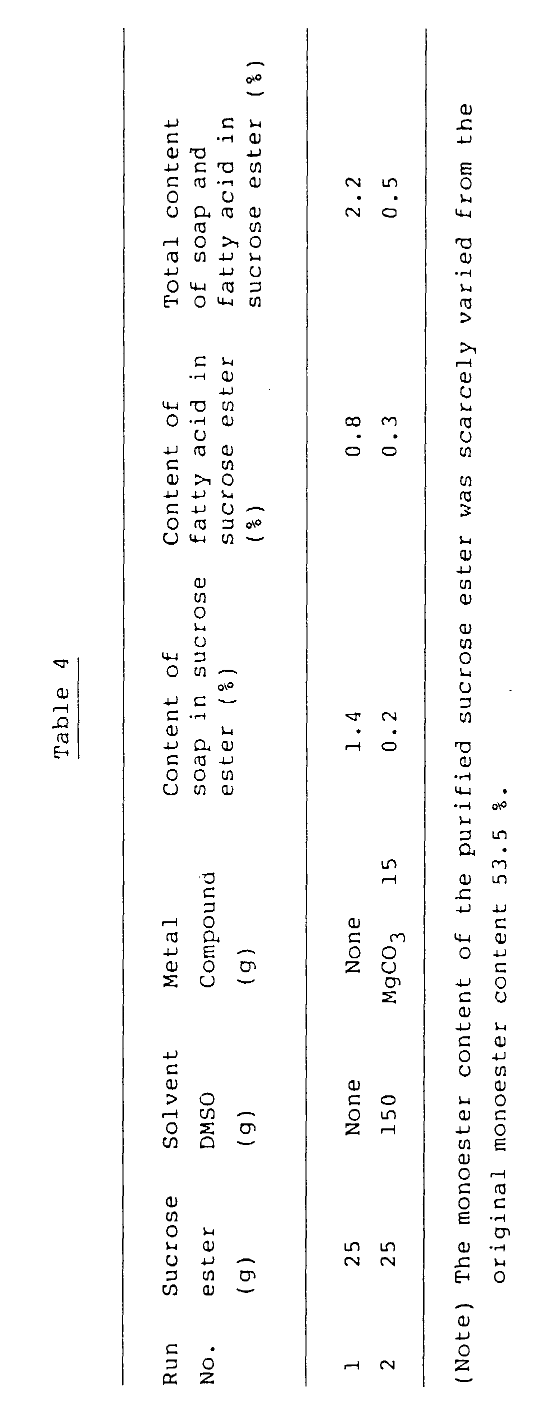

## Claims

1. A process for removing soaps and fatty acids from sucrose fatty acid esters which comprises adding a metal compound selected from the group consisting of calcium oxide, magnesium oxide, calcium hydroxide, magnesium hydroxide and magnesium carbonate to a solution of a sucrose fatty acid ester in an organic solvent having a water content of at most 1.0 % by weight, stirring the solution to precipitate a soap and a fatty acid, which are included in said sucrose fatty acid ester, in the form of a metal soap and separating the resulting precipitate from the solution.

2. The process for claim 1, wherein said organic solvent is a member selected from the group consisting of dimethylsulfoxide, dimethylformamide and propylene glycol.

3. The process of claim 1 or 2, wherein said stirring is carried out at a temperature between ordinary temperature and 100°C for 10 to 40 minutes.

4. The process of claim 1, 2 or 3, wherein said metal compound is used in an amount of 20 to 200 parts by weight per 100 parts by weight of said sucrose fatty acid ester.

5. The process of claim 1, wherein said metal compound is at least one member selected from the group consisting of calcium oxide, magnesium oxide, magnesium hydroxide and magnesium carbonate.

## Patentansprüche

1. Verfahren zur Entfernung von Seifen und Fettsäuren aus Saccharosefettsäureestern, welches das Zugeben einer aus der Calciumoxid, Magnesiumoxid, Calciumhydroxid, Magnesiumhydroxid und Magnesiumcarbonat umfassenden Gruppe gewählten Metallverbindung zu einer Lösung von einem Saccharosefettsäureester in einem organischen Lösungsmittel mit einem Wassergehalt von höchstens 1,0 Gew.-%, das Rühren der Lösung zum Ausfällen einer Seife und einer Fettsäure, welche in dem Saccharosefettsäureester eingeschlossen sind, in der Form einer Metallseife und das Abtrennen des resultierenden Präzipitats von der Lösung umfaßt.

2. Verfahren gemäß Anspruch 1, wobei das organische Lösungsmittel ein aus der Dimethylsulfoxid, Dimethylformamid und Propylenglykol umfassenden Gruppe gewählter Vertreter ist.

3. Verfahren gemäß Anspruch 1 oder 2, wobei das Rühren bei einer Temperatur zwischen normaler Temperatur und 100°C 10 bis 40 Minuten lang durchgeführt wird.

4. Verfahren gemäß Anspruch 1, 2 oder 3, wobei die Metallverbindung in einer Menge von 20 bis 200 Gew.-Teilen pro 100 Gew.-Teile des Saccharosefettsäureesters verwendet wird.

5. Verfahren gemäß Anspruch 1, wobei die Metallverbindung mindestens ein aus der Calciumoxid, Magnesiumoxid, Magnesiumhydroxid und Magnesiumcarbonat umfassenden Gruppe gewählter Vertreter ist.

## Revendications

1. Procédé de séparation de savons et d'acides gras d'esters d'acides gras de saccharose, lequel comprend l'addition d'un composé de métal sélectionné dans le groupe constitué par l'oxyde de calcium, l'oxyde de magnésium, l'hydroxyde de calcium, l'hydroxyde de magnésium et le carbonate de magnésium à une solution d'un ester d'acide gras de saccharose dans un solvant organique ayant une teneur en eau d'au maximum 1,0 % en poids, l'agitation de la solution pour précipiter un savon et un acide gras qui sont inclus dans ledit ester d'acide gras de saccharose, sous la forme d'un savon de métal et la séparation du précipité obtenu de la solution.

2. Procédé selon la revendication 1, où ledit solvant organique est un élément sélectionné dans le groupe constitué par le diméthylsulfoxyde, le diméthylformamide et le propylèneglycol.

3. Procédé selon la revendication 1 ou 2, où ladite agitation est opérée à une température comprise entre la température ambiante et 100°C pendant 10 à 40 min.

4. Procédé selon la revendication 1, 2 ou 3, où ledit composé de métal est utilisé en une quantité de 20 à 200 parties en poids pour 100 parties en poids dudit ester d'acide gras de saccharose.

5. Procédé selon la revendication 1, où ledit composé de métal est au moins un élément sélectionné dans le groupe constitué par l'oxyde de calcium, l'oxyde de magnésium, l'hydroxyde de magnésium et le carbonate de magnésium.
